# EUROPEAN PATENT APPLICATION

(11) **EP 4 640 739 A1**
(43) Date of publication of application: **29.10.2025**
(21) Application number: 23907176.4
(22) Date of filing: 22.12.2023
(51) Int. Cl.: C08G 64/02, A61K 8/891, A61Q 1/12, A61Q 17/04, C08F 299/04, C08F 299/06, C08F 299/08, C08G 77/448

(54) **REACTIVE GROUP-CONTAINING POLYCARBONATE COMPOUND, NEW SILICONE ELASTOMER PARTICLES USING SAME, COSMETIC MATERIAL COMPOSITION, AND OTHER USE APPLICATION**

(30) Priority: 23.12.2022 JP 2022206753
(71) Applicant: Dow Toray Co., Ltd., Tokyo 140-8617 (JP)
(72) Inventor: SUGIURA, Tsunehito, Ichihara-shi Chiba 299-0108 (JP); TANIGUCHI, Hiroko, Ichihara-shi Chiba 299-0108 (JP); TAN, Liyi, Ichihara-shi Chiba 299-0108 (JP); KANZAKI, Yasue, Ichihara-shi Chiba 299-0108 (JP)
(74) Representative: Elkington and Fife LLP
(86) International application number: PCT/JP2023/046102
(87) International publication number: WO 2024/135816

(57) **Abstract**

PROBLEM

To provide a reactive group-containing polycarbonate compound capable of forming silicone elastomer particles by hydrosilylation reaction and radical polymerization reaction, and a method for producing the same. Furthermore, the present invention provides silicone elastomer particles made from the aforementioned compound as a raw material, uses thereof, and a production method thereof.

SOLUTION

A reactive group-containing polycarbonate compound having two or more modified polycarbonate structures in each molecule, expressed by the following structural formula (1):
{where x and y are both numbers in a range of 0 to 18, n is a number in a range of 0 to 30, and Ra is a reactive group selected from a (meth)acryl terminal group and an alkenyl terminal group having 2 to 20 carbon atoms};
novel silicone elastomer particles having a silicon atom crosslinking structure derived from the compound and capable of imparting excellent feel and sensation during use to cosmetic materials; and uses thereof.

## Description

### TECHNICAL FIELD

The present invention provides a reactive group-containing polycarbonate compound that has a specific modified polycarbonate structure containing a reactive group selected from (meth)acryl terminal groups and alkenyl terminal groups in each molecule, and is capable of forming silicone elastomer particles by a hydrosilylation reaction and a radical polymerization reaction, and a method for producing the same. Furthermore, the present invention relates to a novel silicone elastomer particle having a crosslinked structure between silicon atoms derived from the reactive group-containing polycarbonate compound, and capable of imparting excellent texture and feel during use to a cosmetic material. Furthermore, the novel silicone elastomer particles have a crosslinked structure that is active with respect to biodegradability. Therefore, primary particles thereof are expected to have a property of disintegrating with the generation of non-crosslinked siloxane molecules due to a decomposition reaction of microorganisms and the like in the natural world, and thus are expected to behave as biodegradable silicone elastomer particles. Furthermore, the present invention relates to a cosmetic material raw material, cosmetic material composition, organic resin additive, and other applications containing the silicone elastomer particles, as well as to a method of manufacturing the silicone elastomer particles.

### BACKGROUND ART

Silicone elastomer particles are cured from an addition reaction-curable or condensation reaction-curable silicone composition, and the particle diameter and oil absorbency thereof vary depending on the manufacturing method, but are widely used as stress-relieving agents or the like for cosmetic material raw materials and thermoplastic resins. For example, as silicone particles having superior dispersibility, high lipophilicity, and superior storage stability, the present applicant has proposed silicone particles containing an alkylene group having 4 to 20 carbon atoms, which is obtained by curing a crosslinkable composition for forming silicone particles having a low amount of silicon atom-bonded hydrogen atoms per unit mass and containing an alkenyl group having 4 to 20 carbon atoms, such as a hexenyl group or the like, as described in Patent Document 1.

In addition, the applicant has focused on essential issues in conventional silicone elastomer particles. In other words, conventional silicone elastomer particles are formed through a crosslinking reaction of organopolysiloxane raw materials by hydrosilylation reactions or the like. The crosslinked structure is chemically stable, and even if these silicone elastomer particles were to be released into nature, it is undeniable that they would remain in nature without decomposing, at least for a short period of time, just like so-called microplastics. Therefore, there seems to be a latent desire in the market for silicone elastomer particles that perform well enough to smoothly replace or substitute existing silicone elastomer particles and that are expected to be highly biodegradable, in order to reduce risk to the global environment.

In view of this potential market demand, the present applicants have proposed a silicone elastomer particle having a structure crosslinked by a divalent organic group having a partial structure formed by radical polymerization of vinyl acetate as described in Patent Document 2. The silicone elastomer particles can be expected to have a high degree of biodegradability, have suppressed aggregation properties over time as compared with conventional silicone elastomer particles, and provide a smaller average secondary particle diameter. Therefore, these silicone elastomer particles have excellent dispersibility, and have excellent handling workability as a cosmetic material raw material, storage stability, and blending stability in a system.

However, there is still a need for silicone elastomer particles that can impart a feel equal to or better than that of existing silicone elastomer particles and that can be expected to have high biodegradability when used as a cosmetic material raw material.

On the other hand, polycarbonate compounds are synthesized from dimethyl carbonate and diols having hydroxyl groups on both terminals, and are expected to have properties as a biodegradable raw material, in addition to being used as a film-forming material. Furthermore, although Patent Document 3 describes the introduction of a (meth)acrylate functional group into a polycarbonate structure, it does not describe, suggest, nor disclose silicone elastomer particles having the aforementioned polycarbonate structure.

On the other hand, Non-Patent Documents 1 and 2 disclose a reaction of a polycarbonate compound having a polyol terminal structure with an acryloyl chloride or the like, but do not disclose a (meth)acryl-modified polycarbonate compound with a specific structure that can form silicone elastomer particles by a crosslinking reaction between polysiloxane structures or a radical polymerization reaction with a (meth)acryl group-containing organopolysiloxane.

### RELATED ART DOCUMENTS

### PATENT DOCUMENTS

Patent Document 1: International Patent Publication WO2017/191798
Patent Document 2: International Patent Publication WO2022/138346
Patent Document 3: Japanese Unexamined Patent Application 2006-70145

### [NON-PATENT DOCUMENTS]

Non-Patent Document 1: Branching by reactive end groups. II. Synthesis, branching, and melt rheology of (meth)acrylate/pt-butylphenol-coterminated bisphenol a polycarbonates (M.J. Marks et al., Journal of Polymer Science: Part A: Polymer Chemistry, Vol. 38, 2340-2351 (2000) Non-Patent Document 2: Synthesis, crosslinking, and abrasion and weathering properties of (meth)acrylate-terminated bisphenol A polycarbonates (M.J. Marks et al., Journal of Applied Polymer Science / Volume 73, Issue 5, P663-675)

### SUMMARY OF THE INVENTION

### PROBLEM TO BE SOLVED BY THE INVENTION

An object of the present invention is to solve the aforementioned problems, and provide silicone elastomer particles having a crosslinked structure which is active with respect to biodegradability, and can provide texture and feel during use that is equal to or higher than conventional silicone elastomer particles when blended in a cosmetic composition or the like, a reactive group-containing polycarbonate compound having a specific structure and useful as a raw material for synthesis reactions, and a method for producing the same.

Furthermore, an object of the present invention is to provide a cosmetic material raw material, an organic resin additive, and other applications with excellent feel during use, and the like by using the silicone elastomer particles. Furthermore, an object of the present invention is to provide a cosmetic material composition containing the silicone elastomer particles, with excellent feel during use, and the like.

Furthermore, an object of the present invention is to provide: silicone elastomer particles that, in addition to performance equal to or better than conventional silicone elastomer particles, can be expected to be biodegradable, thereby reducing potential risks to the global environment, allowing for industrially sustainable and stable use, and making it possible to promote the material as biodegradable and eco-friendly to users and consumers in general who are concerned about global environmental impact. Also to provide a synthesis material, and a use thereof.

### MEANS FOR SOLVING THE PROBLEM

In order to solve the above problems, the present invention inventors have conducted intensive research and have found that the aforementioned problem can be solved by a reactive group-containing polycarbonate compound having in each molecule 2 or more modified polycarbonate structures expressed by the following structural formula (1): 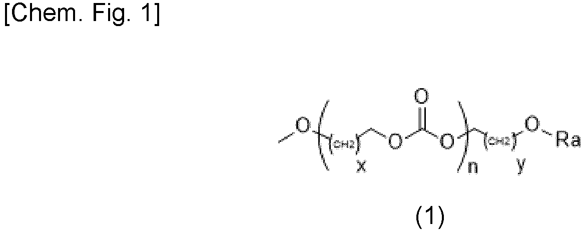
{where x and y are both numbers ranging from 0 to 18, n is a number ranging from 0 to 30, and Ra is a reactive group selected from (meth)acryl terminal groups expressed by -C(=O)-R¹-CR²=CH₂ (R¹ is a chemical bond between CH and C(=O) or a divalent organic group having 0 to 20 carbon atoms, and R² is a hydrogen atom or a methyl group) and alkenyl terminal groups having 2 to 20 carbon atoms};
as well as use thereof as a raw material for silicone elastomer particles.
Thereby the present invention was achieved.
Note that the aforementioned reactive group refers to one or more reactive functional group selected from (meth)acryl terminal groups and alkenyl terminal groups having radical polymerizability or hydrosilylation reactivity, or in other words, the reactive group-containing polycarbonate compound according to the present invention includes both a (meth)acryl-modified polycarbonate compound and an alkenyl-modified polycarbonate compound.

Similarly, the present inventors found that the above problems can be solved by silicone elastomer particles having a structure in which at least two silicon atoms in the silicone elastomer particles are crosslinked by one or more reactions selected from radical polymerization reactions and hydrosilylation reactions of the reactive group-containing polycarbonate compound, more specifically, the radical polymerization reaction of a (meth)acryl-modified polycarbonate compound and the hydrosilylation reaction of an alkenyl-modified polycarbonate compound with a silicon-bonded hydrogen atom, as well as by cosmetic material raw materials, organic resin additives, cosmetic materials, and organic resins containing the same, thus achieving the present invention.

### EFFECT OF THE INVENTION

When the silicone elastomer particles obtained by using the reactive group-containing polycarbonate compound according to the present invention as a raw material are blended in a cosmetic material composition or the like, it is possible to achieve a texture and a feel during use which are equal to or higher than those of conventional silicone elastomer particles. Furthermore, the silicone elastomer particles according to the present invention can be used to provide a cosmetic material raw material, an organic resin additive, and other applications containing the silicone elastomer particles. Furthermore, a cosmetic material composition containing the silicone elastomer particles according to the present invention can provide a cosmetic material with excellent feel during use, and the like.

Furthermore, the silicone elastomer particles of the present invention have a structure crosslinked by a divalent organic group having a partial structure formed by a radical polymerization or hydrosilylation reaction of the reactive group-containing polycarbonate compound between at least 2 silicon atoms constituting the polyorganosiloxane chain in the silicone elastomer particles, and the divalent organic group having this partial structure is active in a biodegradable reaction, and the crosslinked structure formed between silicon atoms in the silicone elastomer particles is at least partially cleaved in a biodegradable environment, and the primary particles of the silicone elastomer particles are designed to have the property of being disintegrated with the generation of a polyorganosiloxane having a non-crosslinked structure. Therefore, the silicone elastomer particles according to the present invention are expected to be biodegradable, which reduces the risk to the global environment, and appeals to users and consumers in general, who are concerned about global environment impact, as an eco-friendly material that can be used with a considerable sense of ease.

### PREFERRED MODE FOR CARRYING OUT THE INVENTION

In the present specification, the term "(meth)acryl" refers to "acryl or methacryl", and when expressed as "(meth)acryl-modified group" indicates that the modifying group (including terminal groups) may be one or both of an acryl modifying group and a methacryl modifying group. Similarly, the term "(meth) acryloxy" refers to "methacryloxy or acryloxy" and "(meth)acryloxy group-containing organic group" indicates either one or both of methacryloxy group-containing organic groups and acryloxy group-containing organic groups.

### [Reactive group-containing polycarbonate compound]

The reactive group-containing polycarbonate compound according to the present invention is, in detail, at least one compound selected from the group consisting of (meth)acryl-modified polycarbonate compounds and alkenyl-modified polycarbonate compounds, and is designed as a reactive raw material for the novel silicone elastomer particles according to the present invention. It is a component that, through its radical polymerization reaction and hydrosilylation reaction with silicon-bonded hydrogen atoms, provides a structure crosslinked within the silicone elastomer particles by a divalent organic group having a specific partial structure between at least two silicon atoms that constitute the polyorganosiloxane chain.

Specifically, the reactive group-containing polycarbonate compound of the present invention has two or more modified polycarbonate structures expressed by the following structural formula (1) in the molecule:

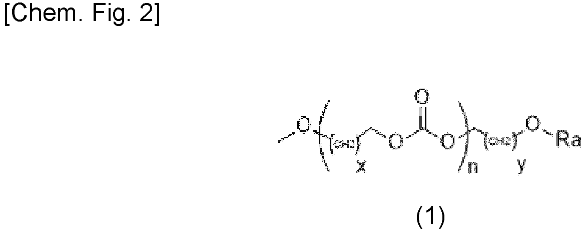

This compound has two or more (meth)acryl-modified groups or alkenyl-modified groups at the terminals, so it is expected that when a crosslinked structure is formed in the silicone elastomer particles by a hydrosilylation reaction or radical polymerization reaction, and the resulting silicone elastomer particles are used as a cosmetic material raw material, the feel of use and texture will not be impaired, and the crosslinked structure formed between the two silicon atoms will have biodegradability.

Here, n in the formula is the number of repeating carbonate units {-O-C(=O)-O-CH₂-(CH₂)x-O-} in the structure, and may be a number in a range of 0 to 30, a number in a range of 0 to 15, or a number in a range of 0 to 10. Furthermore, the number of repeating units, n, may be essential and may be a number in a range of 1 to 30, may be a number in a range of 1 to 15, or may be a number in a range of 1 to 10. The (meth)acryl-modified polycarbonate compounds and alkenyl-modified polycarbonate compounds according to the present invention are designed as crosslinking agents between silicon atoms in the silicone elastomer particles, have a relatively small number of polycarbonate units in each structure, and have none or a relatively small number of repeating units of polycarbonate units in the molecule as a whole, and therefore have the advantage of not significantly impairing the texture and feel during use derived from the organopolysiloxane main chain of the silicone elastomer particles.

In the formula, Ra is a reactive group selected from (meth)acryl terminal groups expressed by -C(=O)-R¹-CR²=CH₂ and alkenyl terminal groups having 2 to 20 carbon atoms. Herein, R¹ is a chemical bond between CH and C(=O) or a divalent organic group with 1 to 20 carbon atoms, and is preferably a simple chemical bond as in "-C(=O)-CH=" or an alkylene group with 1 to 20 carbon atoms expressed by "-C(=O)-CmH₂m-CH=" (m is a number in a range 0 to 20), such as CmH₂m (note that m is 0 when R¹ is a chemical bond between CH and C(=O)). Furthermore, R² is a hydrogen atom or a methyl group, and provides an acryl-modified group or a methacryl-modified group or an alkenyl group.

In the formula, x and y both independently represent the number of methylene groups expressed by CH₂, and are numbers ranging from 0 to 18, preferably from 1 to 18, more preferably from 1 to 10, and particularly preferably from 1 to 5.

The reactive group-containing polycarbonate compound of the present invention is a crosslinking agent between at least two silicon atoms, so it must have at least two reactive groups selected from reactive (meth)acryl-modified groups and alkenyl-modified groups in the molecule. Therefore, the reactive group-containing polycarbonate compound of the present invention is required to have two or more of the above structures in the molecule, and may have 2 to 4 of the above structures. This is because a polycarbonate compound having a polyolic (alcoholic) hydroxyl group terminal which is a precursor of the structure is relatively easily available as a reactive production raw material on an industrial scale.

In the reactive group-containing polycarbonate compound of the present invention, in addition to the fact that the number of repeating polycarbonate units in each structure is relatively small, the sum of the numbers of repeating polycarbonate units in the molecule is preferably in a range of 2 to 20, and may be in a range of 2.5 to 15, or in a range of 3.0 to 12. If the number of repeating carbonate units in the (meth)acryl-modified polycarbonate compound and alkenyl-modified polycarbonate compound molecules exceeds the upper limit, the properties derived from the polycarbonate structure will be strongly reflected in the resulting silicone elastomer particles, which may have an adverse effect on the feel and sensation of use of the cosmetic material or the like.

More specifically, the reactive group-containing polycarbonate compound of the present invention may be a compound expressed by the following structural formula (1-1).

### Structural formula (1-1):

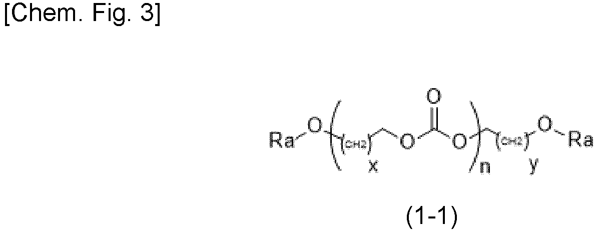

(where Ra is the same group as defined above, and x, y, and n are the same numbers as defined above).

Such a reactive group-containing polycarbonate compound can be obtained by reacting a precursor polycarbonate compound having a polyol terminal structure with a (meth)acryloyl chloride compound and an alkenoyl chloride compound in the presence of a basic catalyst.

Specifically, the reactive group-containing polycarbonate compound according to the present invention can be obtained by:
reacting a polycarbonate compound expressed by the following structural formula (1'): 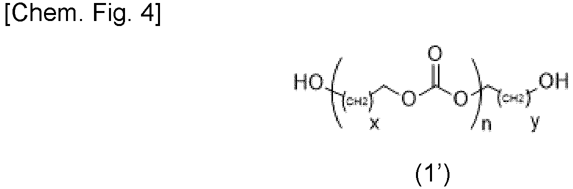
(where x, y, and n are the same numbers as defined above),
   and
a (meth)acryloyl chloride compound expressed by Cl-C(=O)-R¹-CR²=CH₂ (where R¹ and R² are the same groups as defined above),
in the presence of a basic catalyst.
In the structural formula (1') expressing the polycarbonate compound having a polyol terminal structure, n is preferably in a range of 1 to 15.

The reaction ratio of the polycarbonate compound having a polyol terminal structure to the (meth)acryloyl chloride compound and the alkenoyl chloride compound is such that the amount (number of moles) of the (meth)acryloyl chloride compound and the alkenoyl chloride compound is in a range of 1 equivalent to a slight excess relative to the amount (number of moles) of the polyol terminal structure (-OH) of the polycarbonate compound.

The basic catalyst that can be used in the reaction is not particularly limited, and may be an alkali metal salt of an inorganic base such as sodium hydroxide, potassium hydroxide, calcium hydroxide, sodium carbonate, potassium carbonate or sodium hydrogen carbonate; an amine compound such as triethylamine, pyridine, dimethylaminopyridine, or triazabicyclodecene; or a nitrogen-containing heterocyclic compound.

Examples of organic solvents that can be used in the reaction include ketones such as acetone, methyl ethyl ketone, methyl isobutyl ketone and cyclohexanone; amides such as formamide, acetamide, N-methyl-2-pyrrolidone (NMP), N,N-dimethylformamide, and dimethylacetamide; halogenated hydrocarbons such as methylene chloride, chloroform, 1,2-dichloroethane, chlorobenzene, bromobenzene, dichlorobenzene, benzotrifluoride and hexafluoro-2-propanol; sulfoxides such as dimethyl sulfoxide (DMSO), diethyl sulfoxide and benzylphenyl sulfoxide; ethers such as diethyl ether, diisopropyl ether, dibutyl ether, tetrahydrofuran (THF), dioxane, 1,2-dimethoxyethane and cyclopentylmethyl ether; esters such as ethyl acetate; nitriles such as acetonitrile and benzonitrile; aromatic hydrocarbons such as benzene, toluene and xylene; and mixtures of two or more of these.

In this reaction, one or more types of polymerization inhibitors may be included in the system, in order to inhibit the synthesized reactive group-containing polycarbonate compound from undergoing further radical polymerization reaction. Examples include one or more types selected from hindered phenol-based polymerization inhibitors, hydroquinone-based polymerization inhibitors, and catechol-based polymerization inhibitors.

The reaction conditions should be appropriately selected based on the synthesis amount, the reaction apparatus, and the like, but the (meth)acryloyl chloride compound and the alkenoyl chloride compound are added dropwise while stirring a mixed solution containing the polycarbonate compound, the basic catalyst, and the optional polymerization inhibitor under a flow of an inert gas such as nitrogen. Note that after the reaction is completed, it is particularly preferable to separate and purify the desired reactive group-containing polycarbonate compound by distilling off the unnecessary organic solvent under reduced pressure.

### [Silicone elastomer particles]

The silicone elastomer particles of the present invention, applications thereof including cosmetic material raw materials in particular, a manufacturing method thereof, as well as a cosmetic material composition and an organic resin (including paint and coating agent) containing the same will be described in detail below.

The silicone elastomer particles of the present invention have a structure in which at least two silicon atoms in the silicone elastomer particles are crosslinked by one or more reactions selected from radical polymerization reactions of the reactive group-containing polycarbonate compound described above and hydrosilylation reactions of silicon-bonded hydrogen atoms.

More specifically, the silicone elastomer particles of the present invention are obtained by a crosslinking reaction selected from a radical polymerization reaction and a hydrosilylation reaction, and each of these have the following structural features.

### [Radical polymerization-reactive silicone elastomer particles]

The silicone elastomer particle is obtained by radically polymerizing an organopolysiloxane having three or more silicon-bonded radical reactive functional groups such as a (meth)acryloxy group-containing organic group bonded to a silicon atom in the molecule and a (meth)acryl-modified polycarbonate compound in the presence of a radical polymerization initiator, and has a crosslinked structure formed between at least two silicon atoms in the silicone elastomer particles by a radical polymerization reaction between the terminal (meth)acryl-modified group of the (meth)acryl-modified polycarbonate compound and the silicon-bonded radically reactive functional group.

### [Hydrosilylation-reactive silicone elastomer particles]

The silicone elastomer particle is obtained by subjecting an organopolysiloxane (= organohydrogenpolysiloxane) having three or more silicon-bonded hydrogen atoms in the molecule and the alkenyl-modified polycarbonate compound to a hydrosilylation reaction in the presence of a hydrosilylation reaction catalyst, and at least two silicon-silicon bonds in the silicone elastomer particles have a crosslinked structure formed by a hydrosilylation reaction (addition reaction) between an alkenyl-modified group of the alkenyl-modified polycarbonate compound and a silicon-bonded hydrogen atom.

The silicone elastomer particles of the present invention preferably further contain a polyorganosiloxane structure expressed by:

-(R₂SiO)ₘ-

(where R represents an unsubstituted alkyl group having 1 to 20 carbon atoms, an alkyl group having 1 to 20 carbon atoms substituted with a halogen atom, an aryl group having 6 to 22 carbon atoms, or a hydroxyl group, and m is a number ranging from 1 to 1000).
This is a linear polysiloxane structure derived from component (A), described later, which provides the silicone elastomer particles with an appropriate degree of hardness and flexibility.

Industrially, R preferably independently represents a methyl group or phenyl group, and m is more preferably a number ranging from 50 to 800, and more preferably 75 to 750.

The silicone elastomer particles according to the present invention are preferably obtained by curing crosslinking reactive silicone emulsified particles by a crosslinking reaction. Particularly preferably, the silicone elastomer particles of the present invention are defined by the production process, and are silicone elastomer particles obtained by performing in water a crosslinking reaction of crosslinkable silicone emulsified particles obtained by emulsifying in water a crosslinkable silicone composition crosslinkable by one or more reactions selected from radical polymerization reactions and hydrosilylation reactions with a silicon-bonded hydrogen atom, the composition containing at least:
(A) one or more type of reactive organopolysiloxane selected from the following component (a1) and component (a2);
   (a1) an organopolysiloxane having at least three organic groups containing a (meth)acryloxy group of at least one type selected from organic groups containing a methacryloxy group and organic groups containing an acryloxy group in each molecule;
   (a2) an organopolysiloxane having at least three silicon atom-bonded hydrogen atoms per molecule;
(B) a reactive group-containing polycarbonate compound; and
(C) one or more curing agent selected from radical polymerization initiators and hydrosilylation reaction catalysts.

Furthermore, silicone elastomer particles obtained via such a manufacturing process may further improve the appearance, spreadability, and tactile sensation of the cosmetic material, particularly when used as a cosmetic material raw material, and particles obtained via this manufacturing method tend to be more suitable for solving the problem of the present invention. Thus, one suitable form for achieving the technical effect of the present invention can be and is suitably defined by the manufacturing process.

The silicone elastomer particles according to the present invention are not particularly limited in terms of the average primary particle diameter thereof, but from the perspective of imparting a smooth texture and feel during use to the cosmetic material, not causing appearance defects and the like, storage stability and blending stability as a cosmetic material raw material, and the like, the average primary particle diameter measured by a laser diffraction scattering method is preferably within a range of 0.5 to 20 µm, more preferably within a range of 0.5 to 15 µm. Note that the particle diameter of the silicone elastomer particles can be controlled according to the crosslinking reactive silicone emulsified particles and crushing/classification process of the resulting silicone elastomer particles.

The shape of the silicone elastomer particles according to the present invention includes, for example, spherical, regular spherical shape, elliptical, and irregular shapes, and in particular, spherical and regular spherical shapes are preferred. Spherical silicone elastomer particles can be easily obtained by the method described later, in which the particles are prepared in the form of an aqueous suspension and dried using a vacuum dryer, hot air circulation oven, or spray dryer.

Furthermore, in the present invention, the crosslinking reactive silicone composition used to form the silicone elastomer particles is preferably within a range of 10 to 80 when cured in a sheet form, as measured by a JIS A hardness tester as defined in JIS K6301. If the JIS-A hardness of the rubber sheet measured by curing the crosslinking reactive silicone composition into a sheet form is within the range above, the resulting silicone elastomer particles will have sufficiently low aggregation and will tend to have exceptional flowability, dispersibility, smoothness, silkiness, and a soft texture. Furthermore, by selecting the JIS-A hardness described above, it is possible to design or predict to some extent the feel during use, texture, and handling workability when added to a cosmetic material, and to improve stress relaxation properties when added to an organic resin. Note that when the silicone elastomer particles according to the present invention are used as a cosmetic material raw material or stress relief agent or the like for organic resins, silicone elastomer particles having a JIS-A hardness in a range of 30 to 80, and particularly 50 to 80, described above are particularly preferably used.

Optionally, the silicone elastomer particles of the present invention may further contain a structure in which some or all of the surface thereof is covered with one or more types selected from organopolysiloxane resins, silica, and other silicone elastomer particles. Such coating may be expected to further reduce aggregation, control oil absorbency, improve texture, and the like.

Optionally, the silicone elastomer particles of the present invention may be mesoporous structures with micropores.

Optionally, the silicone elastomer particles of the present invention may contain an oil agent that is liquid at 40°C. The oil agent can be easily included in the silicone elastomer particles by emulsifying together in the crosslinking reactive silicone composition described later, and the inclusion of the oil agent may be expected to further reduce aggregation, control oil absorbency, improve texture, and the like.

Optionally, the silicone elastomer particles of the present invention may further have a structure in which at least two silicon atoms configuring the particles are crosslinked by a silalkylene group having 2 to 20 carbon atoms formed by a hydrosilylation reaction involving an alkenyl group having 2 to 20 carbon atoms. These structures can be easily achieved by using an alkenyl group-containing organopolysiloxane, organohydrogen polysiloxane, and hydrosilylation reactive catalyst in combination. Furthermore, for example, by using an alkenyl group having 4 or more carbon atoms, such as a hexenyl group or the like, further reduction of aggregation, control of oil absorbency, and improvement of texture may be expected due to the combination of the silalkylene structure. However, if biodegradability is a primary objective, the silicone elastomer particles of the present invention are preferably substantially free of structures containing a silalkylene group.

[Crosslinking reactive silicone composition for Use in Formation of Silicone Elastomer Particles]

The silicone elastomer particles of the present invention can be obtained by crosslinking (curing) a crosslinking reactive silicone composition containing the following components by at least one type of reaction selected from a radical polymerization reaction and a hydrosilylation reaction to the silicon atom bonded hydrogen atom.
(A) at least one type of reactive organopolysiloxane selected from the following component (a1) and component (a2);
   (a1) an organopolysiloxane having at least three organic groups containing a (meth)acryloxy group of at least one type selected from organic groups containing a methacryloxy group and organic groups containing an acryloxy group in each molecule;
   (a2) an organopolysiloxane having at least three silicon atom-bonded hydrogen atoms per molecule;
(B) reactive group-containing polycarbonate compound;
(C) one or more curing agents selected from radical polymerization initiators and hydrosilylation reaction catalysts.

Note that the crosslinking (curing) reaction is one or more type of reaction selected from radical polymerization reactions and hydrosilylation reactions, and these reactions may be allowed to proceed simultaneously, but from the viewpoint of reaction control, selecting either one to form the silicone elastomer particles is preferable. In other words, the composition may be a composition containing the following two reaction models and components.

### [Composition for forming radical polymerization-reactive silicone elastomer particles]

The crosslinkable silicone composition contains:
(a1) an organopolysiloxane having at least three organic groups containing at least one type of (meth)acryloxy group selected from organic groups containing a methacryloxy group and organic groups containing an acryloxy group in each molecule;
(B) a (meth)acryl-modified polycarbonate compound, which is a reactive group-containing polycarbonate compound; and
(c1) a photoradical polymerization initiator.

### [Composition for forming hydrosilylation-reactive silicone elastomer particles]

A crosslinked reactive silicone composition, containing:
(a2) an organopolysiloxane having at least three silicon atom-bonded hydrogen atoms per molecule;
(B) the alkenyl-modified polycarbonate compound, which is a reactive group-containing polycarbonate compound; and
(c2) a hydrosilylation reaction catalyst.

Component (a1) is an organopolysiloxane having at least three organic groups containing a (meth)acryloxy group in a molecule, the structure of which is not particularly limited and may be one or more type of structure selected from linear, cyclic, reticulate, or partially branched linear structures. However, a linear organopolysiloxane is particularly preferred. The viscosity of component (a) is preferably a viscosity that allows the crosslinkable composition above to be dispersed in water. Specifically, it is preferably in a range of 20 to 100,000 mPa·s at 25°C, and particularly preferably in a range of 20 to 10,000 mPa·s.

From the perspective of texture, dispersibility and handling workability of the silicone elastomer particles, component (a1) is preferably a linear organopolysiloxane in which the amount of the dimethylsiloxane units expressed by the formula: (CH₃)₂SiO is 80 mol% or more of all siloxane units other than the siloxane units at molecular terminals. Similarly, from the perspective of improving the oil absorbency of the resulting silicone elastomer particles and the like, a cyclic or chain organopolysiloxane with a low degree of polymerization (degree of polymerization of 3 to 20) may be removed from component (a1) beforehand by stripping or the like.

Furthermore, when the component (a1) is a linear organopolysiloxane, the silicone elastomer particles according to the present invention, when placed in a biodegradable environment, have an advantage that when the silicone elastomer particles are broken down by cleavage of the crosslinked structure, they are more likely to break down into non-crosslinkable, linear organopolysiloxanes, thus reducing the environmental impact and environmental risk.

Component (a1) must have at least three or more organic groups containing a (meth)acryloxy group on average in a molecule to form a crosslinked structure in a radical reaction with component (B). **If** only two or fewer organic groups containing a (meth)acryloxy group are present in a molecule on average, a sufficient crosslinked structure may not be formed, and practical silicone elastomer particles may not be obtained.

More specifically, the organic group containing a (meth)acryloxy group is a (meth)acryloxy group bonded to a silicon atom via a divalent organic group, and one or more functional group expressed by the following is exemplified.

-R²-O-C(=O)-C(R³)=CH₂

{where R² represents an alkylene group having 1 to 20 carbon atoms or a divalent linking group expressed by

(CH₂)p-Si(CH₃)₂-O-Si(CH₃)₂-(CH₂)q

(p and q in the formula are each a number ranging from 1 to 20), and R³ represents a hydrogen atom or a methyl group.}

The alkylene group, which is R² in the formula, may industrially be an alkylene group having 2 to 10 carbon atoms, and examples include propylene groups, butylene groups, hexylene groups, and the like. Furthermore, a divalent linking group expressed by (CH₂)ₚ-Si(CH₃)₂-O-Si(CH₃)₂-(CH₂)_{q} is a divalent linking group having a siloxane converter structure, and industrial examples include linking groups where p and q are each independently a number from 3 to 6.

Suitably, component (a1) is preferably a linear organopolysiloxane expressed by the following structural formula.

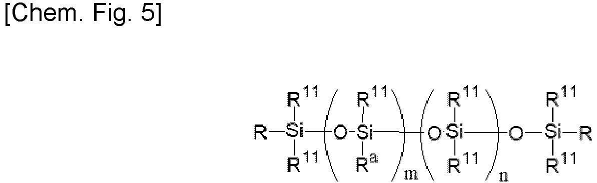

In Formula (1), each R¹¹ is independently an unsubstituted or halogen atom-substituted alkyl group (for example, a methyl group, or the like) having 1 to 20 carbon atoms, an aryl group (for example, a phenyl group, or the like) having 6 to 22 carbon atoms, or a hydroxyl group, and a methyl group or a phenyl group is preferable from an industrial point of view. R^{a} represents an organic group containing a (meth)acryloxy group, and is particularly preferably a (meth)acryloxy group bonded to a silicon atom by the aforementioned alkylene group or divalent linking group with a siloxane converter structure. R independently is a group represented by R¹¹ or Ra. m is a number of 1 or more, and n is a number of 1 or more. However, component (a) contains at least three organic groups containing a (meth)acryloxy group represented by R^{a} in each molecule, and therefore, when m = 1, R must both be R^{a}. In other words, the linear organopolysiloxane expressed by the structural formula above has an organic group containing a (meth)acryloxy group represented by R^{a} at one end site and a side chain site, a side chain site only, or both end sites and a side chain site of a siloxane molecule thereof, and preferably contains at least three organic groups containing a (meth)acryloxy group in a molecule.

m+n is the degree of siloxane polymerization of linear organopolysiloxane molecules excluding end siloxane structures. From the perspective of handling workability as a raw material and the ability to break down into fine linear siloxane molecules during emulsification biodegradation, m + n is preferably within a range of 10 to 800, more preferably 20 to 600, and particularly preferably 30 to 500. Furthermore, the viscosity of component (a) is particularly preferably a number between 20 and 10,000 mPa·s at 25°C.

Component (a2) is an organopolysiloxane component that is crosslinked by component (B) by a hydrosilation reaction, is characterized by having at least three silicon-bonded hydrogen atoms in each molecule, and the bonding positions of the hydrogen atoms in the molecules are not particularly limited.

Examples of an organic group other than a hydrogen atom that is bonded to a silicon atom contained by component (a2), include an alkyl group such as a methyl group, an ethyl group, a propyl group, a butyl group, or an octyl group, and a methyl group is preferable. Examples of the molecular structure of the organohydrogen polysiloxane of component (a2), include straight-chain, branched-chain, and branched-cyclic, or a combination of one or more thereof. Note that the number of silicon-bonded hydrogen atoms in a molecule is the average value of all molecules.

In particular, when the component (a2) is a linear organopolysiloxane (organohydrogenpolysiloxane), the silicone elastomer particles according to the present invention, when placed in a biodegradable environment, have an advantage that when the silicone elastomer particles are broken down by cleavage of the crosslinked structure, they are more likely to break down into non-crosslinkable, linear organopolysiloxanes, thus reducing the environmental impact and environmental risk.

The viscosity of component (a2) at 25 °C is 1 to 1,000 mPa-s, preferably 5 to 500 mPa-s. This is because if the viscosity of component (b) at 25 °C is less than 1 mPa-s, component (a2) is easily volatilized from the crosslinkable composition containing it, and if the viscosity exceeds 1,000 mPa-s, the curing time of the crosslinkable composition containing such component (a2) may become longer or may cause curing failure. Such component (a2) is not particularly limited, and examples include, dimethylsiloxane-methylhydrogensiloxane copolymer capped at both molecular terminals with trimethylsiloxy groups, dimethylsiloxane-methylhydrogensiloxane copolymer capped at both molecular terminals with dimethylhydrogensiloxy groups, dimethylpolysiloxane capped at both molecular terminals with dimethylhydrogensiloxy groups, methylhydrogenpolysiloxane capped at both molecular terminals with trimethylsiloxy groups, cyclic methylhydrogenpolysiloxane, and cyclic methylhydrogensiloxane-dimethylsiloxane copolymer.

Here, the value of H/Alk, which is the molar ratio (= reaction ratio in the hydrosilylation reaction) of the carbon-carbon double bond (Alk) in the alkenyl terminal groups in component (B) to the silicon atom-bonded hydrogen atom amount (H) in component (a2) is preferably in a range of 0.7 to 1.2. The lower limit of said H/Alk is preferably 0.80 or more, 0.85 or more, 0.90 or more, or 0.95 or more, and the upper limit is 1.15 or less, and preferably, 1.10 or less, or 1.05 or less. If the upper limit of H/Alk exceeds the aforementioned value, unreacted silicon-bonded hydrogen atoms tend to remain after the reaction; conversely, if the upper limit of H/Alk is less than the aforementioned value, unreacted component (B) and (meth)acryl terminal groups thereof tend to remain after the reaction. Since these are curing reactive groups, if a large amount of them remains in the particles, they may cause crosslinking reactions between particles over time, resulting in aggregation and poor dispersion of the resulting oil-containing silicone elastomer particles, and if reactive hydrogen atoms remain, they may cause the generation of flammable hydrogen gas over time. Particularly suitably, when the value of H/Alk is in a range of 0.9 to 1.1, particularly around 1.0, the curing reactive groups are completely consumed and the crosslinking reaction is terminated, and the aggregation over time between particles can be effectively suppressed.

Component (B) is a reactive group-containing polycarbonate compound (specifically, (meth)acryl-modified polycarbonate compound or alkenyl-modified polycarbonate compound), and provides a crosslinking agent for component (A) which provides the silicone elastomer particles of the present invention with a characteristic crosslinked structure, and a radical polymerizable monomer component that forms a polymer or copolymer structure through radical polymerization. The crosslinked portions, whether of the hydrosilylation reaction type or the radical polymerization type, do not significantly impair the feel during use and texture of the resulting silicone elastomer particles, are active in biodegradable reactions, and in a biodegradable environment, the crosslinked structures formed between the silicon atoms in the silicone elastomer particles are at least partially cleaved, and the primary silicone elastomer particles have the property of disintegrating with the generation of non-cross-linked polyorganosiloxane structures. Herein, if component (A) is a linear polyorganosiloxane as described above, the biodegradable reaction facilitates the breakdown of the silicone elastomer particles into linear polyorganosiloxane molecules, which are broken down into fine liquid components, not solid powders with minute particle diameter sizes, as in macroplastics. Therefore, it is expected to have little impact or burden on the global environment, as it is unlikely to cause problems of bioaccumulation through the food chain or accumulation/deposition in the environment.

The amount of component (B) used must be selected as follows in accordance with the type of component (A) and the crosslinking reaction for obtaining the silicone elastomer particles of the present invention.

When silicone elastomer particles are formed by the radical polymerization reaction of the aforementioned component (a1) and component (B), the mass ratio of the amount of component (B) to the amount of the (meth)acryloxy group-containing organic group in component (a1) is in a range of 0.5 to 50, preferably a range of 1 to 20, preferably 3 to 15, and particularly preferably 5 to 10. If the amount of component (B) used is within a range described above, a crosslinked structure derived from (meth)acryl-modified polycarbonate compounds with an appropriate average length can be obtained between the polyorganosiloxane structures, so that the particles can achieve a smooth surface state with moderate hardness and low tack, and the feel during use and texture can be improved. On the other hand, if the amount of component (B) used is less than the lower limit above, crosslinking may be insufficient. If the amount of component (B) used exceeds the upper limit above, emulsion breakdown and the like are likely to occur during a curing reaction, and thus silicone elastomer particles may not be obtained.

When the silicone elastomer particles are formed by the hydrosilation reaction between components (a2) and (B), the amount of component (B) is preferably designed such that the molar ratio of the carbon-carbon double bonds (Alk) in the alkenyl terminal groups to the silicon-bonded hydrogen content (H) of component (a2), or in other words, H/Alk, is within the above range.

Component (C) is a curing agent and is selected from (c1) a radical polymerization initiator and (c2) a hydrosilylation reaction catalyst, depending on the selection of component (A) and the reaction system.

Component (c1) is a radical initiator and is a component for promoting a radical polymerization reaction or radical copolymerization reaction of components (a1) and (B) described above. A conventionally known compound commonly used in radical polymerization methods is used as the radical initiator, and specific examples include 2,2'-azobis(isobutyronitrile), 2,2'-azobis(2-methylbutyronitrile), 2,2'-azobis(2,4-dimethylvaleronitrile), and other azo-based compounds; benzoyl peroxide, lauroyl peroxide, tert-butylperoxy benzoate, tert-butylperoxy-2-ethylhexanoate, tert-hexylperoxy-2-ethylhexanoate, and other organic peroxides; and potassium persulfate, sodium persulfate, ammonium persulfate, and other persulfates. One type of these radical initiators may be used alone, or two or more types may be mixed and used together.

The amount of component (c1), which is a radical initiator, used is preferably within a range of 0.1 to 5 parts by mass relative to 100 parts by mass of the total of components (a1) and (B) above. In particular, when component (c1) is a water-soluble persulfate such as potassium persulfate or the like, component (c1) has an advantage of being extremely easy to add and react when crosslink reacting in water crosslinking reactive silicone emulsified particles, which are obtained by emulsifying a crosslinking reactive silicone composition in water via a radical polymerization reaction. Furthermore, upon completion of the radical polymerization reaction, it is particularly desirable to add aminomethylpropanediol or the like in a range of 0.1 to 5 parts by mass, for the purpose of stopping the reaction and neutralizing the solution by pH adjustment.

The timing of adding component (c1) to the crosslinkable composition can be selected according to the method of forming the silicone elastomer particles, and may be in the form of adding to the composition in advance, or in the form of supplying component (a1) or component (B) from a different spray line, then adding component (c2) to one of the components, and then mixing during spraying. The silicone elastomer particles in the present invention are preferably formed via an aqueous suspension, which is formed via emulsification into water, and component (c1) may be added in advance to the crosslinking reactive silicone composition, or an emulsion containing component (c1) may be added separately to the water.

A chain transfer agent can be optionally added during the polymerization reaction of the crosslinking reactive silicone composition above. Specific examples of the chain transfer agent include mercapto compounds such as 2-mercaptoethanol, butyl mercaptan, n-dodecyl mercaptan, 3-mercaptopropyl trimethoxysilane, polydimethylsiloxanes having a mercaptopropyl group, and the like; and halides such as methylene chloride, chloroform, carbon tetrachloride, butyl bromide, 3-chloropropyl trimethoxysilane, and the like.

Component (c2) is a hydrosilylation reaction catalyst, which promotes the addition reaction (hydrosilylation reaction) between the carbon-carbon double bond in the alkenyl terminal group present in the above crosslinkable composition and a silicon atom-bonded hydrogen atom. Examples of preferred hydrosilylation reaction catalysts are hydrosilylation reaction catalysts containing platinum metal, such as platinic acid chloride, alcohol-modified platinic acid chloride, olefin complexes of platinic acid chloride, complexes of platinic acid chloride with ketones, complexes of platinic acid chloride with vinyl siloxane, platinum tetrachloride, platinum fine powder, platinum supported on an alumina or silica carrier, platinum black, olefin complexes of platinum, alkenylsiloxane complexes of platinum, carbonyl complexes of platinum, and thermoplastic organic resin powders such as methyl methacrylate resin, polycarbonate resin, polystyrene resin, and silicone resin containing these platinum-based catalysts. In particular, platinum alkenylsiloxane complexes such as a complex of platinum chloride with divinyltetramethyldisiloxane, a complex of platinum chloride with tetramethyltetravinylcyclotetrasiloxane, a platinum divinyltetramethyldisiloxane complex, and a platinum tetramethyltetravinylcyclotetrasiloxane complex can be preferably used. Note, as the catalyst for promoting the hydrosilylation reaction, a non-platinum-based metal catalyst such as iron, ruthenium, iron/cobalt, or the like may be used.

The amount of component (c2) added to the crosslinkable composition should be a catalytic quantity, and usually, an amount in which the amount of the platinum-based metal contained by component (c2) is in a range of 1 to 1,000 ppm relative to the total mass of the crosslinkable composition described above is preferred, and an amount in a range of 5 to 500 ppm is even more preferred. Note that the amount of platinum metal in the silicone elastomer particles may be reduced by the method proposed by the present inventors in Japanese Unexamined Patent Application 2014-122316.

The timing of adding component (c2) to the crosslinkable composition can be selected according to the method of forming the silicone elastomer particles, and may be in the form of adding to the composition in advance, or in the form of supplying component (a2) or component (B) from a different spray line, then adding component (c2) to one of the components, and then mixing during spraying. The oil-containing silicone elastomer particles of the present invention are preferably formed via an aqueous suspension, which is formed via emulsification into water, and component (c2) may be added in advance to the crosslinking reactive silicone composition, or an emulsion containing component (c2) may be added separately to the water.

The crosslinkable silicone composition described above may include a curing retarder represented by a hydrosilylation reaction inhibitor. Examples of such curing retarders are acetylene compounds, enyne compounds, organic nitrogen compounds, organic phosphorus compounds, and oxime compounds. Specific compounds include 2-methyl-3-butyn-2-ol, 3,5-dimethyl-1-hexyn-3-ol, 3-methyl-1-pentyn-3-ol, 2-phenyl-3-butyn-2-ol, and 1-ethynyl-1-cyclohexanol (ETCH) and other alkyne alcohols; 3-methyl-3-trimethylsiloxy-1-butyne, 3-methyl-3-trimethylsiloxy-1-butyne, 3,5-dimethyl-3-trimethylsiloxy-1-hexyne, 3-methyl-3-penten-1-yne, and 3,5-dimethyl-3-hexene-1-yne and other enyne compounds; 1-ethynyl-1- trimethylsiloxycyclohexane, bis(2,2-dimethyl-3-butynoxy)dimethylsilane, methyl(tris(1,1-dimethyl-2-propynyloxy))silane, 1,3,5,7-tetramethyl-1,3,5,7-tetravinylcyclotetrasiloxane; and 1,3,5,7-tetramethyl-1,3,5,7-tetrahexenylcyclotetrasiloxane and other alkenylsiloxanes. The amount added is within a range of 0.001 to 5 mass parts per 100 mass parts of component (a), but can be designed as appropriate depending on the type of curing retarder used, the characteristics of the hydrosilylation reaction catalyst used, and the amount used.

The crosslinking reactive silicone composition above may contain one or more polymerization inhibitors from the perspective of preventing unintended side reactions and the like. For example, one or more types selected from hindered phenol-based polymerization inhibitors, hydroquinone-based polymerization inhibitors, and catechol-based polymerization inhibitors may be included. The amount used can be selected as appropriate, but the total concentration of the polymerization inhibitor is preferably 50 ppm by mass or less, and more preferably 30 ppm by mass or less, with respect to the sum of components (A) to (C) above.

The crosslinking reactive silicone composition may include a component other than the components above to the extent that the technical effects of the present invention are not impaired. For example, the composition may include: n-hexane, cyclohexane, n-heptane, or other aliphatic hydrocarbons; toluene, xylene, mesitylene, or other aromatic hydrocarbons; tetrahydrofuran, dipropyl ether, or other ethers; an organic solvent such as acetone, methyl ethyl ketone, methyl isobutyl ketone, other ketones, or the like; a phenolic antioxidant, quinone antioxidant, amine antioxidant, phosphorus antioxidant, phosphite antioxidant, sulfur-based antioxidant, thioether antioxidant, or other antioxidant; a triazole light stabilizer, benzophenone light stabilizer, or other light stabilizer; a phosphate ester flame retardant, halogen flame retardant, phosphorus flame retardant, antimony flame retardant, or other flame retardant; one or more antistatic agents including cationic surfactants, anionic surfactants, non-ionic surfactants, and the like; a dye; a pigment; or the like.

The silicone elastomer particles of the present invention may optionally further have: (A) a structure in which part or all of a surface thereof is coated by one or more substance selected from organopolysiloxane resins, silica, and other silicone elastomer particles; (B) a mesoporous structure; (C) a structure containing an oil agent that is liquid at 40°C; and (D) a structure crosslinked by a silalkylene group having 2 to 20 carbon atoms, and a combination of arbitrary components providing these structures may be used.

### [Hardness of silicone elastomer]

Although the hardness of the silicone elastomer particles cannot be measured directly, the hardness can be measured indirectly by curing the crosslinkable silicone composition used to form the silicone elastomer particles serving as a raw material thereof. Specifically, the crosslinking reactive silicone composition is cured in a sheet form without emulsifying in water, and the hardness of the silicone elastomer sheet can be measured by a JIS A hardness tester as defined in JIS K6301. The hardness of the silicone elastomer according to the present invention varies depending on the type of the crosslinkable silicone composition, the amount of components (a)/(b) used, and the crosslink density, but is preferably within a range of 10 to 80. In addition, the preferred hardness is as described above.

### [Formation of Silicone Elastomer Particles and Manufacturing Method Thereof]

The silicone elastomer particles of the present invention can be provided by a method including a step of curing crosslinking reactive silicone emulsified particles, which are obtained by emulsifying in water with the crosslinking silicone composition used to form the silicone elastomer particles above, in the presence of (C) a curing agent to obtain spherical silicone elastomer particles.

More specifically, the silicone elastomer particles according to the present invention can and preferably are prepared using a manufacturing method including the following steps (I) and (II).
Step (I): a step of forming crosslinkable emulsified particles, by emulsifying in water,
   (A) at least one type of reactive organopolysiloxane selected from the aforementioned component (a1) and component (a2);
   (B) a reactive group-containing polycarbonate compound (specifically, at least one modified polycarbonate compound selected from the aforementioned (meth)acryl-modified polycarbonate compound and alkenyl modified polycarbonate compound); and
   (C) one or more curing agents selected from radical polymerization initiators and hydrosilylation reaction catalysts;
Step (II): a step of curing the crosslinking reactive silicone emulsified particle obtained in step (I) in the presence of (C) a curing agent to obtain silicone elastomer particles.

The crosslinkable silicone composition used to form the silicone elastomer particles can be uniformly mixed using a mixer or other mechanical force.

In this method, silicone elastomer particles can be obtained by emulsifying and curing the aforementioned crosslinkable silicone composition in a surfactant aqueous solution. In addition, the particle diameter can be easily adjusted by adjusting the emulsion particle diameter. Examples of these surfactants include nonionic, anionic, cationic, betaine types, as well as water soluble polymers such as polyvinylalcohols and the like. The particle diameter of the resulting silicone elastomer particles varies depending on the type and content of the surfactant. In order to prepare silicone elastomer particles having a small particle diameter, the amount of the surfactant added is preferably within a range of 0.5 to 50 parts by mass with respect to 100 parts by mass of the crosslinkable silicone composition.

An emulsifier is preferably used to uniformly disperse the crosslinking silicone composition in water in the form of crosslinking reactive silicone emulsified particles. Examples of the emulsifying machine include a homomixer, a paddle mixer, a Henschel mixer, a homo-disper, a colloid mill, a propeller agitator, a homogenizer, an in-line continuous emulsifier, an ultrasonic emulsifier, and a vacuum kneading machine.

The aqueous dispersion of the crosslinking reactive silicone emulsified particles prepared by the method above can then be heated or left at room temperature to cure the crosslinking reactive silicone emulsified particles in the aqueous dispersion to prepare an aqueous dispersion of silicone elastomer particles. When such an aqueous dispersion is heated, the heating temperature is preferably 100°C or lower from the perspective of hydrosilylation reactivity or radical polymerization reactivity, and is particularly preferably 10 to 95°C. Furthermore, examples of a method of heating the aqueous dispersion containing the crosslinking reactive silicone emulsified particles include a method of directly heating the water-based dispersion, and a method of adding the aqueous dispersion to hot water. The crosslinking reaction causes the liquid crosslinking reactive silicone emulsified particles to cure in water, forming a aqueous dispersion of silicone elastomer particles.

The resulting silicone elastomer particles of the present invention can be used as-is as an aqueous dispersion (aqueous suspension). In particular, the aqueous suspension form may be and is preferably used in cosmetic material raw materials and the like. When added to a cosmetic material that uses an aqueous solution as a dispersion medium (such as hair cosmetic materials and the like), the silicone elastomer particles may be easily and uniformly dispersed to achieve a desired performance and feel during use by being added as an aqueous dispersion containing the silicone elastomer particles of the present invention.

Suitably, the silicone elastomer particles according to the present invention can be isolated by removing water from the aqueous dispersion of silicone elastomer particles. The method of removing water from the aqueous dispersion includes, for example, drying using a vacuum dryer, a hot air circulation oven, or a spray dryer. Note that the heating and drying temperature of the spray dryer must be set appropriately based on the heat resistance, crosslinking temperature, and the like of the silicone elastomer particles. Note that in order to prevent secondary aggregation of the resulting microparticles, the temperature of the silicone elastomer particles is preferably controlled to be equal to or lower than the glass transition temperature thereof. The silicone elastomer particles thus obtained can be recovered by a cyclone, a bag filter, or the like. Note that as a pretreatment for this operation, the dispersion may be concentrated by a method of heating and dehydration, filtration separation, centrifugation, decantation, or the like, and if necessary, the dispersion may be washed with water.

The silicone elastomer particles of the present invention may be surface treated, if necessary, to further improve the aggregation suppression effect of the silicone elastomer particles of the present invention. Furthermore, surface treatment with another known hydrophilic or hydrophobic treatment agent or the like may be applied. Optionally, the resulting silicone elastomer particles may be further coated with silica or other inorganic microparticles, silicone resin, or the like, in whole or in part on the surface thereof, as described above. Furthermore, the resulting silicone elastomer particles may be crushed or pulverized by a mechanical force if necessary, or classified using a known technique.

### [Cosmetic Material Raw Material and Cosmetic Material Composition]

The silicone elastomer particles of the present invention are useful as cosmetic material raw materials, and when blended in a cosmetic composition or the like, the particles are flexible and improve the feel and texture of the cosmetic materials, and the like, and provide outstanding handling workability, storage stability, and blending stability in systems as a cosmetic material raw material.

In particular, the silicone elastomer particles of the present invention are superior to known silicone particles in terms of feel during use and texture, have a higher degree of freedom in formulation design, do not absorb oily components over time when formulated in cosmetic materials, do not cause thickening or changes in texture, and when applied to the skin or hair, suppress greasy or sticky feel, provide a soft and moist feel by spreading smoothly, increase the feeling of compatibility with the skin, and the like. In addition, when the silicone elastomer particles of the present invention are used in combination with a UV protection component, the UV protection effect of the cosmetic material may be improved without impairing the texture and feel during use of the cosmetic material as compared with other powders or existing silicone elastomer particles.

Furthermore, the silicone elastomer particles of the present invention are active with respect to biodegradable reactions while providing performance that is equal or superior to conventionally known silicone elastomer particles. Moreover, a crosslinked structure formed between silicon atoms in the silicone elastomer particle is at least partially cleaved in a biodegradable environment, and primary particles of the silicone elastomer particles have a property of disintegrating with the generation of a polyorganosiloxane with a non-crosslinked structure. Therefore, the silicone elastomer particles are a low risk and low impact material for the global environment. Furthermore, use is possible as a replacement to conventionally known silicone elastomer particles, making it extremely versatile.

The cosmetic material composition containing the silicone elastomer particles of the present invention are not particularly limited in type, and examples include products such as soaps, body shampoos, facial cleansing creams, and other cleaning cosmetic materials; toners, creams/milky lotions, packs, and other base cosmetic products; powders, foundation, and other base makeup cosmetic materials; lipstick, cheek rouge, eye shadow, eyeliners, mascara, and other facial cosmetic materials; nail polish and other makeup cosmetic materials; shampoos, hair rinses, hairdressing material, hair growth promoters, hair dye, and other hair cosmetic materials; perfume and eau de cologne, and other aromatic cosmetic materials; toothpastes; bath agents; and hair removal agents, shaving lotions, antiperspirants/deodorants, sunscreen agents, and other special cosmetic materials. Examples of the dosage forms of these cosmetic material compositions include aqueous liquid, oil-based liquid, emulsion, cream, foam, semi-solid, solid, and powder. These cosmetic material compositions can also be used by spraying.

In these cosmetic material compositions, the amount of the silicone elastomer particles described above is preferably within a range of 0.5 to 99.0 mass% in the cosmetic material composition, and particularly preferably within a range of 1.0 to 95 mass%. This is because if the amount of the silicone elastomer particles described above exceeds the upper limit of the above range, the effect as a cosmetic material is lost, and if the amount is below the lower limit of the above range, feel during use and the like of the cosmetic material composition and the like is less likely to be improved.

With respect to cosmetic material compositions (in particular, each formulation example) containing silicone particles (silicone rubber powder and the like) or silicone composite particles, which have been proposed in Patent Document 1 (Japanese Unexamined Patent Application H07-316014), Patent Document 2 (International Patent Publication WO2017/191798), and Patent Document 3 (Japanese Unexamined Patent Application H02-243612) as well as Japanese Unexamined Patent Application 2011-105663, Japanese Unexamined Patent Application 2011-168634, Japanese Unexamined Patent Application 2011-102354, and Japanese Unexamined Patent Application 2014-122316 described above, the silicone elastomer particles of the present invention can be used in place of a part or all of these silicone-based particles, and there are cases where the feel during use as well as the production efficiency of the cosmetic material compositions proposed in these Patent Documents can be further improved. Note that it goes without saying that examples of cosmetic material compositions containing silicone particles (silicone rubber powder or the like) or silicone composite particles that can be blended with the silicone elastomer particles of the present invention are not limited to the above, and formulations may be designed in which some or all of silicone particle components in commercially available cosmetic materials are replaced by the silicone elastomer particles of the present invention, using a technique common to a person of ordinary skill in the art.

Furthermore, the silicone elastomer particles of the present invention can be applied to replace some or all of these silicone-based particles with respect to the applications and formulations of cosmetic material compositions disclosed in the aforementioned Patent Documents and the like, and the use therein is encompassed within the scope of the invention of the present application. As an example, the silicone elastomer particles of the present invention may be and are preferably used in combinations of arbitrary components, such as cosmetic product media (aqueous media or oil-based media), oil-based media (including oil agents and volatile oil agents), water, colorants, pigments, ultraviolet light blocking components, alcohols, water-soluble polymers, film-forming agents, oil agents, oil-soluble gelling agents, organically modified clay minerals, surfactants, resins, salts, moisturizers, preservatives, antimicrobial agents, antioxidants, pH adjusters, chelating agents, refreshing agents, anti-inflammatory agents, skin brightening agents (such as whitening agents, cell activators, skin roughness improvement agents, blood circulation promoters, skin astringents, and anti-seborrheic agents), vitamins, amino acids, nucleic acids, hormones, inclusion compounds, bioactive substances, medicament active components, perfumes, and the like, by selecting a method or quantitative range similar to those disclosed in Patent Document 2 (International Patent Publication WO2017/191798).

In particular, the silicone elastomer particles of the present invention have superior feel during use, texture, handling workability, storage stability, dispersibility, and high oil absorption properties that are equal or superior to conventionally known silicone particles, silicone composite particles coated with silsesquioxane, and silicone particles containing an oil agent. Therefore,
(1) cosmetic material compositions and formulations containing oil-based media such as oil agents and the like (oil-based cosmetic material raw materials);
(2) cosmetic material compositions and formulations containing lipophilic ultraviolet light blocking components (such as octyl paramethoxycinnamate and the like); and
(3) cosmetic material compositions and formulations containing inorganic powders such as colorants, pigments, or the like,
can provide a particularly favorable appearance, feel of use, and the like. These specific formulations are further described in detail in the Examples.

In addition thereto, the silicone elastomer particles of the present invention can be easily designed for aqueous dispersions. Therefore, in aqueous cosmetic material compositions and formulations, the silicone elastomer particles provide excellent formulation design freedom and blending stability, and thus can achieve a suitable feel during use. These specific formulations are further described in detail in the Examples.

The cosmetic material of the present invention can be easily manufactured by simply uniformly mixing the cosmetic product raw material of the present invention and other cosmetic product raw materials as described above. As mixing means, various mixing and kneading devices normally used in the manufacture of cosmetic products can be used. Examples of such devices include a homomixer, a paddle mixer, a Henschel mixer, a homodisper, a colloidal mixer, a propeller agitator, a homogenizer, an in-line continuous emulsifier, an ultrasonic emulsifier, and a vacuum kneading machine.

### [Organic Resin Additives and Organic Resins, Paints, and Coating Agents]

The silicone elastomer particles of the present invention are also extremely useful as an organic resin additive due to the properties described above. Specifically, the silicone elastomer particles of the present invention have superior uniform dispersibility in organic resins and, if desired, excellent stress relief properties, and the like. In addition, the particles have very excellent handling workability and storage stability because aggregation is less likely to occur even after long-term storage. Furthermore, a member, paint film, or coating film obtained by curing an organic resin containing the silicone elastomer particles has improved flexibility (including the softness of a coating layer), durability, and adhesion and conformability to the substrate, is particularly pliable, and has superior thermal shock resistance. Therefore, it is extremely useful as a highly functional organic resin, paint, or coating agent for use in electronic materials.

### [Organic Resin]

A curable organic resin composition or a thermoplastic resin is suitably exemplified as an organic resin containing the silicone elastomer particles of the present invention. Of these, curable resins are suitable for electronic materials such as semiconductor substrates and the like. More specifically, examples of the curable organic resin composition include, phenolic resin, formaldehyde resin, xylene resin, xylene-formaldehyde resin, ketone-formaldehyde resin, furan resin, urea resin, imide resin, melamine resin, alkyd resin, unsaturated polyester resin, aniline resin, sulfone-amide resin, silicone resin, epoxy resin, and copolymer resins of these resins, and two or more of these curable resins can be combined. In particular, the curing resin is preferably at least one type selected from the group consisting of an epoxy resin, a phenolic resin, an imide resin, and a silicone resin. The epoxy resin can be any compound containing glycidyl or alicyclic epoxy groups, and examples include o-cresol novolac epoxy resins, phenol novolac epoxy resins, biphenyl epoxy resins, bisphenol A epoxy resins, bisphenol F epoxy resins, dicyclopentadiene epoxy resins, naphthalene epoxy resins, anthracene epoxy resins, naphthol aralkyl epoxy resins, polyvinylphenol epoxy resins, diphenylmethane epoxy resins, diphenylsulfone epoxy resins, triphenolalkane epoxy resins, cresol-naphthol co-condensation epoxy resins, bisphenylethylene epoxy resins, fluorene epoxy resins, stilbene epoxy resins, spiro-coumarone epoxy resins, norbornene epoxy resins, terpene epoxy resins, phenolcyclohexane epoxy resins, halogenated epoxy resins, imide-group-containing epoxy resins, maleimide-group-containing epoxy resins, allyl group-modified epoxy resins, and silicone-modified epoxy resins. Examples of this phenolic resin include a polyvinylphenol type, a phenol novolac type, a naphthol type, a terpene type, a phenol dicyclopentadiene type, a phenol aralkyl type, a naphthol aralkyl type, a triphenol alkane type, a dicyclopentadiene type, a cresol naphthol co-condensation type, and a xylene-naphthol co-condensation type. An example of a silicone resin is an epoxy-modified silicone resin generated by a reaction between an epoxy resin and a silanol group or a silicon-bonded alkoxy group in the silicone resin. Examples of the curing mechanisms of such curable resins are thermal curing, high energy beam curing such as ultraviolet light, radiation, and the like, moisture curing, condensation reaction curing, and addition reaction curing. The properties of such curable resins at 25 °C are not limited, and may be in either a liquid state or a solid state that softens upon heating.

Another optional component such as a curing agent, curing promoter, filler, photosensitizer, higher fatty acid metal salt, ester wax, plasticizer, or the like can be added to the organic resin containing the silicone elastomer particles of the present invention. Examples of curing agents include: organic acids such as carboxylic acids, sulfonic acids, and the like and anhydrides thereof; organic hydroxy compounds; organic silicon compounds having a silanol group, an alkoxy group, or a halogeno group; and primary or secondary amino compounds, and two or more types can be combined. Examples of the curing promoter include: tertiary amine compounds, organic metal compounds such as aluminum, zirconium, and the like; organophosphorus compounds such as phosphine and the like; other heterocyclic amine compounds, boron complex compounds, organic ammonium salts, organic sulfonium salts, organic peroxides, and catalysts for hydrosilylation. Examples of these fillers include: fibrous fillers such as glass fiber, asbestos, alumina fiber, ceramic fiber composed of alumina and silica, boron fiber, zirconia fiber, silicon carbide fiber, metal fiber, polyester fiber, aramid fiber, nylon fiber, phenolic fiber, natural animal and plant fiber, and the like; powdered fillers such as fused silica, precipitated silica, fumed silica, calcined silica, zinc oxide, calcined clay, carbon black, glass beads, alumina, talc, calcium carbonate, clay, aluminum hydroxide, barium sulfate, titanium dioxide, aluminum nitride, silicon carbide, magnesium oxide, beryllium oxide, Kaolin, mica, zirconia, and the like. Two or more of these can be combined. In the case of epoxy resins, it is particularly preferable to include an amine curing agent.

The silicone elastomer particles of the present invention may be added as an additive to a thermoplastic resin other than those described above, and may be used as a modifier of physical properties such as surface lubricants, stress relief agents, and the like, or modifiers of optical properties such as light scattering agents and the like. The type of thermoplastic resin is not particularly limited, and may be at least one polymer selected from a group consisting of: polycarbonate resins; polyester resins; polyether resins; polylactic acid resins; polyethylene, polypropylene, ethylene-propylene copolymers, and other polyolefin resins; polystyrene resins; styrene copolymers; tetrafluoroethylene and other fluorine-based polymers; polyvinyl ethers; and cellulose-based polymers, or a composite resin containing a combination of these. The silicone resin coated silicone elastomer particles of the present invention can be uniformly dispersed in these thermoplastic resins (including master batches) using a mixing device such as a biaxial or single-axis extruder, a kneader mixer, or the like, and may be molded into a desired shape, such as a film form or the like, for use.

The added amount of the silicone elastomer particles of the present invention may be selected as appropriate according to the physical properties required of the organic resin, but is generally within a range of 0.1 to 30 parts by mass with respect to 100 parts by mass of the organic resin, and may be within a range of 0.5 to 10 parts by mass. The reason is that if the amount of the particles added is less than the lower limit, the performance such as stress relief properties for the resin or the like may become insufficient, and the pliability and thermal shock resistance of the resulting cured organic resin product may decrease, and in particular, the thermal shock resistance tends to decrease after moisture absorption. On the other hand, if the amount exceeds the upper limit, the organic resin or the paint/coating agent after blending may become thickened and the handling workability may decrease, and the mechanical properties of the resulting organic resin cured product tend to decrease.

Furthermore, the silicone elastomer particles of the present invention are superior in stress relief when added to an organic resin, and thus may be added to an epoxy resin or the like for printed wiring boards to form a prepreg. Furthermore, a copper foil containing filler particles for a printed wiring board provided with a resin layer containing the silicone elastomer particles of the present invention on one side of the copper foil may be formed and used for a copper clad laminate (CCL) application.

### [Paints, Coating Agents]

Examples of paints and coating agents containing the silicone elastomer particles of the present invention include ambient temperature curing types, ambient temperature drying types, and heat curing types, with examples based on the properties thereof including aqueous types, oil-based types, and powdered types, and examples based on the vehicle resin including polyurethane resin paint, butyral resin paint, long oil phthalate resin paint, alkyd resin paint, amino alkyd resin paint made up of amino resin and alkyd resin, epoxy resin paint, acryl resin paint, phenol resin paint, silicone modified epoxy resin paint, silicone modified polyester resin paint, and silicone resin paint.

The added amount of the silicone elastomer particles of the present invention can be selected as appropriate according to the physical properties required for the paint/coating agent, but in order to impart uniform and soft matting properties to the resulting coating film, the added amount is preferably within a range of 0.1 to 150 parts by mass with respect to 100 mass parts of solid content of the paint, more preferably within a range of 0.1 to 100 parts by mass, even more preferably 0.1 to 50 parts by mass, and particularly preferably 0.1 to 20 parts by mass. If the amount of the particles added is less than the lower limit, performance such as matting, adhesion, stress relief properties, and the like of the coating film may be insufficient. If the amount of the particles exceeds the upper limit, the organic resin and the paint/coating agent after blending may become thickened and the handling workability may decrease.

The paints and coating agents containing the silicone elastomer particles of the present invention may contain: alcohols such as methanol, ethanol, and the like; ketones such as methyl ethyl ketone, methyl isobutyl ketone, and the like; esters such as ethyl acetate, butyl acetate, cellosolve acetate, and the like; amides such as N,N-dimethylformamide and the like; olefins such as hexane, heptane, octane, and the like; organic solvents such as toluene, xylene, and other aromatic hydrocarbons; known inorganic fillers such as reinforcing silica and the like; organic fillers; curing promoters; silane coupling agents; carbon black and other pigments; dyes; antioxidants; thickeners containing a macromolecular compound; flame retardants; and weather resistance imparting agents.

### [As Eco-Friendly Material]

As described above, unlike conventional non-biodegradable thermoplastic resin particles and silicone particle materials, the silicone elastomer particles of the present invention are expected to be biodegradable in a biodegradable environment, where crosslinked structures formed between silicon atoms within the silicone elastomer particles are at least partially cleaved, and primary particles of the silicone elastomer particles have a property of disintegrating with the generation of a polyorganosiloxane with a non-crosslinked structure. Therefore, use is possible as an "eco-friendly" cosmetic material raw material with low environmental burden and environmental risk that complies with regulations on microplastics and the like, and appeal as a biodegradable and "eco-friendly" material is expected for users and consumers who are concerned about global environmental impact.

### EXAMPLES

With the reactive group-containing polycarbonate compound according to the present invention (specifically, a (meth)acryl-modified polycarbonate compound and an alkenyl-modified polycarbonate compound), silicone elastomer particles using the compound as a raw material, and a method for producing the particles will be described in detail with reference to Examples and Comparative Examples. However, the present invention is not limited only to these examples. The viscosity in the examples is the value at 25°C. The properties of each silicone particle were measured as follows. Note that in the examples and the like, unless otherwise specified, silicone particles is a generic term for particles made of a silicone cured product (cured silicone particles), and does not include emulsions.

### [Average Primary Particle Diameter of Emulsion Particles]

The emulsion before the addition of the radical polymerization initiator or before the addition of the hydrosilylation catalyst was measured using a laser diffraction particle diameter distribution analyzer (LS-230 from Beckman Coulter), and the median diameter (particle diameter corresponding to 50% of the cumulative distribution, 50% particle diameter) was used as the average particle diameter.

### [Average Secondary Particle Diameter of Silicone Particles (Powder)]

Using ethanol as a dispersion medium, the particle diameter of the cured silicone particles was measured with a laser diffraction particle diameter distribution analyzer (Mastersizer 3000 from Malvern Panalytical), and the median diameter of the cured silicone particles in ethanol (particle diameter corresponding to 50% of the cumulative distribution, D90, µm) and the arithmetic dispersion (particle diameter distribution SD, µm²) values were obtained. For the measurement sample, cured silicone particles (1 g) and ethanol (100 mL) were dispersed in a 300 mL cup using stirring blades and an ultrasonic vibrator.

Component (A) used in the examples and comparative examples is as follows. Note that "Me" refers to "methyl group".
(a1) Methacryl-modified silicone polymer (viscosity 1524 mPas at 25°C) expressed by the following structural formula: 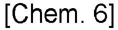 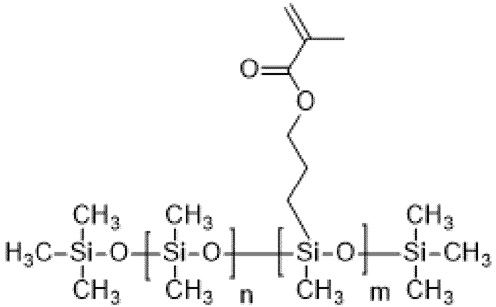
(m = 4, n = 267 in the formula).
(See "Synthesis Example")
(a2) Dimethylsiloxane/methylhydrogensiloxane copolymer terminated at both molecular terminals with a trimethylsiloxy group, expressed by structural formula Me₃SiO(Me₂SiO)₄₇-(Me(H)SiO)₁₅-SiMe₃

### [Synthesis Example]

92.07 parts by weight of dodecamethylcyclosiloxane and 0.01 parts by weight of MEHQ (hydroquinone monomethyl ether, polymerization inhibitor), and 5.88 parts by weight of 3-methacryloxypropylmethyldimethoxysilane were placed in a 4-neck separable flask. The mixture was heated and stirred while blowing nitrogen gas, and when the temperature reached 50°C, 0.05 parts by weight of trifluoromethanesulfonic acid and 1.09 parts by weight of water were added. After reacting at 65°C for 1 hour, the liquid temperature was heated to 70°C. Furthermore, the pressure was reduced to 100 mmHg to remove the methanol produced as a by-product for about 1 hour. Thereafter, 0.90 parts by weight of hexamethyldisiloxane was added and the reaction was carried out for 3 hours. After the reaction, ammonia gas was bubbled to neutralize the trifluoromethanesulfonic acid, and the generated salt was removed by filtration. The filtrate was subjected to reduced pressure processing at 150°C for 3 hours to remove volatile components. C, Si-NMR analysis revealed that (a1) methacryl-modified silicone polymer having 267 dimethylsiloxane units and 4 methacryl group-introduced siloxane units was obtained, which had a viscosity of 1524 mPas.

### [Example 1: Undecenoyl-modified polycarbonate compound b1]

A four-necked separable flask equipped with a reflux condenser was charged with 37.55 parts by weight of DMC (dimethyl carbonate), 52.6 parts by weight of 1,4-butanediol, 9.85 parts by weight of 1,6-hexanediol, and 2536 ppm of DMAP (4-dimethylaminopyridine). The mixture was heated and stirred while bubbling nitrogen gas, and when the liquid temperature inside the flask reached 90°C, the mixture was refluxed for 1 hour. Thereafter, the outlet of the reflux condenser was opened to allow distillation, and when distillation ceased, the pressure was reduced to 2 mmHg, and the reaction was carried out for 5 hours. After the reaction, the pressure was returned to normal and the mixture was cooled to room temperature. After cooling, 6.25 parts by weight of Kyoward 700PL (manufactured by Kyowa Chemical Industry Co., Ltd.: synthetic aluminum silicate) was added and the mixture was stirred for 1 hour. Kyoward 700PL was removed by filtration to obtain a clear liquid polymer. H-NMR analysis revealed that the following diol polycarbonate (PC2) was obtained. 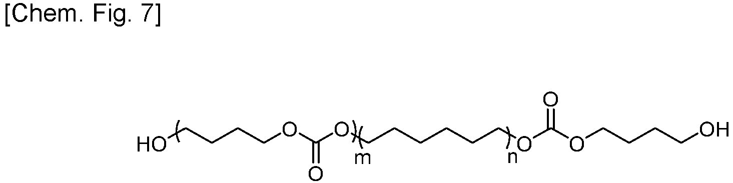 (In the formula, m + n ≧1)
A four-necked separable flask was charged with 11.86 parts by weight of the obtained (PC2), 48.06 parts by weight of chloroform, and 16.6 parts by weight of potassium carbonate. The mixture was stirred while bubbling nitrogen gas, and 23.48 parts by weight of undecenoyl chloride was added dropwise. The reaction was carried out while cooling so that the temperature did not exceed 30°C. After the dropwise addition was completed, the reaction was carried out for about 5 hours and then left overnight. The reaction mixture was filtered, the liquid portion was collected, and the chloroform was removed under reduced pressure. The clear liquid polymer that was obtained was found by H-NMR analysis to be a polycarbonate (b1) modified with an undecenoyl group at the terminus and having the following structure.

### (b1) Structural formula:

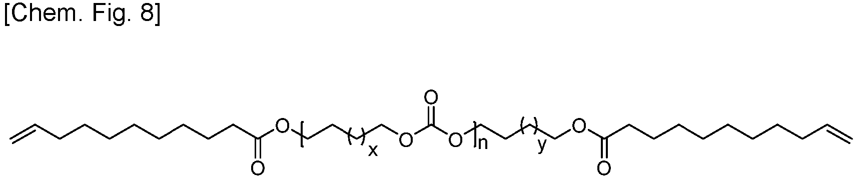

(In the formula, x = 1 or 3, y = 1 or 3, n ≧ 1)

### [Synthesis Example 2: Acryl-modified polycarbonate compound b2]

The same operations as in Example 1 were performed except for using 20.9 parts by weight of (PC2) of Example 1, 46.35 parts by weight of chloroform, 22.12 parts by weight of potassium carbonate, 63 ppm of MEHQ (hydroquinone monomethyl ether, polymerization inhibitor), and 10.63 parts by weight of acryloyl chloride to obtain a polycarbonate ((meth)acryl-modified polycarbonate compound) (b2) having the following structure terminated with an acryl group.

### (b2) Structural formula:

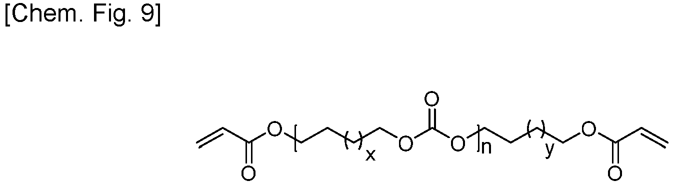

(In the formula, x = 1 or 3, y = 1 or 3, n ≧ 1)

### [Examples 3 to 4, Comparative Example 1: Production of polycarbonate elastomer particles]

Examples 3 and 4 below are production examples of the silicone elastomer particles obtained using the aforementioned undecenoyl-modified polycarbonate compound and (meth)acryl-modified polycarbonate compound as raw materials. Note that Comparative Example 1 is non-silicone-based polymer particle obtained using only a (meth)acryl-modified polycarbonate compound as a raw material.

### [Example 3: Silicone elastomer particle No. 1 (hydrosilylation reaction type)]

The organohydrogenpolysiloxane as component (a2) and the undecenoyl-modified polycarbonate compound as component (b1) were mixed uniformly at room temperature in a mass ratio of 53:47. Thereafter, this composition was dispersed in an aqueous solution at 25°C that was composed of 30 parts by mass of purified water and 0.5 parts by mass of polyoxyethylene alkyl (C12-14) ether. After the mixture was uniformly emulsified using a colloid mill, and 526 parts by mass of purified water was added for dilution to prepare the emulsion. Next, an isopropyl alcohol solution of platinum chloride (an amount of which platinum metal set to 10 ppm by mass in this composition) was added to the emulsion as an aqueous dispersion with polyoxyethylene alkyl (C12-14) ether and pure water, and the emulsion was stirred. Subsequently, the silicone rubber was let stand at 60°C for 6 hours to prepare a uniform aqueous suspension of elastomer particles. The aqueous suspension was then filtered and the residue was dried in an oven at 70°C for 5 hours to obtain silicone elastomer particle No. 1. The average primary particle size and average secondary particle size of the resulting silicone elastomer particles were 4.05 µm and 18.6 µm, respectively.

### [Example 4: Silicone elastomer particles No. 2 (radical polymerization type)]

The methacryl-modified silicone polymer as component (a1) and the (meth)acryl-modified polycarbonate compound (b2) were mixed uniformly at room temperature at a mass ratio of 50:50. Next this composition was dispersed in an aqueous solution at 25°C containing 0.23 parts by mass of GOHSENOL EG-05C (manufactured by Mitsubishi Chemical: polyvinyl alcohol), 0.47 parts by mass of GOHSENOL EG-18P (manufactured by Mitsubishi Chemical: polyvinyl alcohol), and 46 parts by mass of pure water, the resulting mixture was uniformly emulsified using a colloid mill, and then 526 parts by mass of pure water was added to dilute the mixture to prepare an emulsion. After heating in a 1 L flask to 70°C, an aqueous solution of 0.5 g potassium persulfate (manufactured by Sigma-Aldrich) dissolved in 9.5 g water was added dropwise over one minute. The emulsion was subjected to radical polymerization at 70°C for 3 hours, the temperature was further increased to continue the reaction at 80°C for 2 hours, and then 0.8 g of aminomethylpropanediol was then added to end the reaction, thereby preparing a uniform aqueous suspension of silicone elastomer particles. The aqueous suspension was then filtered and washed with 200 mL of ethanol and 100 mL of acetone. The residue was dried in an oven at 70°C for 5 hours to obtain silicone elastomer particles No. 2. The average primary particle size and average secondary particle size of the resulting silicone elastomer particles were 5.04 µm and 53.0 µm, respectively.

### [Comparative Example 1 (radical polymerization type)]

Non-silicone polymer particles were obtained in the same manner as in Example 5, except that a polyorganosiloxane component was not used, and 100 mass parts of only (b2) (meth)acryl-modified polycarbonate compound No. 1 was used. The average primary particle size and average secondary particle size of the obtained particles were 2.80 µm and 259 µm, respectively.

The average primary and secondary particle diameters of each particle obtained from the above Examples 3 and 4 and Comparative Example 1 are summarized in Table 1 below.

**[Table 1]**

| | Average primary particle diameter (µm) | Average secondary particle diameter µm) |
|---|---|---|
| Silicone elastomer particle No. 1 | 4.05 | 18.6 |
| Silicone elastomer particle No. 2 | 5.04 | 53.0 |
| Particles of Comparative Example 1 | 2.80 | 259 |

### [Cosmetic Material Formulation Example]

The following are formulation examples of cosmetic materials of the present invention that can contain silicone elastomer particles, which is one aspect of the present invention. However, the present invention is not limited thereto.

### [Examples 5 and 6 and Comparative Examples 2 and 3]

Panelists compared and evaluated the feel during use of loose powder in which the silicone elastomer particles in the compositions listed in the following Table 3 were used.

### Evaluation of Tactile Sensation

The slipperiness of the samples applied to inner forearms of 18 panelists was evaluated using the criteria in Table 2 below.

**[Table 2]**

| Evaluation results | Evaluation indicators |
|---|---|
| Good | 12 or more of 18 respondents said the slipperiness was favorable |
| △ | 7 to 11 of 18 respondents said the slipperiness was favorable |
| Poor | 6 or fewer of 18 respondents said the slipperiness was favorable |

**[Table 3]**

| Phase | Component | Product name and supplier | COMPARATIVE EXAMPLE 2 | EXAMPLE 5 | EXAMPLE 6 | COMPARATIVE EXAMPLE 3 |
|---|---|---|---|---|---|---|
| A | Silicone elastomer particle No. 1 | (Example 3) | | 10 | | |
| | Silicone elastomer particle No. 2 | (Example 4) | | | 10 | |
| | Particles of Comparative Example 1 | (Comparative Example 1) | | | | 10 |
| | Phenyl trimethicone | DOWSIL^{™} 556 Cosmetic Fluid | 6 | 6 | 6 | 6 |
| B | Talc | JA-46R available from ASADA MILLING CO., LTD. | 76 | 76 | 76 | 76 |
| | Silylated silica | DOWSIL^{™} VM-2270 Aerogel Fine Particle | 2 | 2 | 2 | 2 |
| | Kaolin | | 1.5 | 1.5 | 1.5 | 1.5 |
| | Titanium oxide | SI Titanium CR-50 available from Miyoshi Kasei, Inc. | 3.92 | 3.92 | 3.92 | 3.92 |
| | Iron oxide yellow | SI-YELLOW-LLXLO available from Miyoshi Kasei, Inc. | 0.46 | 0.46 | 0.46 | 0.46 |
| | Iron oxide red | SA-Bengara Cloisonne available from Miyoshi Kasei, Inc. | 0.09 | 0.09 | 0.09 | 0.09 |
| | Iron oxide black | SA-Black BL-100 available from Miyoshi Kasei, Inc. | 0.02 | 0.02 | 0.02 | 0.02 |
| Evaluation of tactile sensation | | | Poor | Good | Good | △ |

### (Method of preparation)

1. Phase A is mixed.
2. Phase B is mixed.
3. Phases A and B are stirred until uniform.

As shown in Table 3, loose powder using the silicone elastomer particles of the present invention (Examples 3 and 4) was evaluated to have favorable slipperiness, as well as loose powder, as compared to no addition products (Comparative example 2) or products with other particles (Comparative Example 3).

### [Examples 7 and 8 and Comparative Examples 4, 5, and 6]

The panelists compared and evaluated the feel during use of water-in-oil sunscreen agents in which the silicone elastomer particles in the compositions listed in Table 5 were used.

### Evaluation of Tactile Sensation

The 18 panelists evaluated the spreadability and white residue when the samples were applied to inner forearms using the criteria in Table 4 below.

**[Table 4]**

| Evaluation results | Evaluation Indicators |
|---|---|
| Good | 12 or more of 18 persons responded favorable |
| △ | 7 to 11 of 18 persons responded favorable |
| Poor | 6 or fewer of 18 persons responded favorable |

**[Table 5]**

| Phase | Component | Product name and supplier | COMPARATIVE EXAMPLE 4 | COMPARATIVE EXAMPLE 5 | EXAMPLE 7 | EXAMPLE 8 | COMPARATIVE EXAMPLE 6 |
|---|---|---|---|---|---|---|---|
| A | Dimethicone | XIAMETER^{™} PMX-200 2cs | 8.55 | 5.55 | 5.55 | 5.55 | 5.55 |
| | Silicone emulsifiers | DOWSIL^{™} ES-5612 Formulation Aid | 4 | 4 | 4 | 4 | 4 |
| | Dimethicone crosspolymer | DOWSIL^{™} EP-9610 Cosmetic Powder | | 3 | | | |
| | Silicone elastomer particle No. 1 | (Example 3) | | | 3 | | |
| | Silicone elastomer particle No. 2 | (Example 4) | | | | 3 | |
| | Particles of Comparative Example 1 | (Comparative Example 1) | | | | | 3 |
| B | Titanium oxide | SI Titanium CR-50 available from Miyoshi Kasei, Inc. | 10.5 | 10.5 | 10.5 | 10.5 | 10.5 |
| | Iron oxide yellow | SI-YELLOW-LLXLO available from Miyoshi Kasei, Inc. | 1.23 | 1.23 | 1.23 | 1.23 | 1.23 |
| | Iron oxide red | SA-Bengara Cloisonne available from Mivoshi Kasei, Inc. | 0.25 | 0.25 | 0.25 | 0.25 | 0.25 |
| | Iron oxide black | SA-Black BL-100 available from Mivoshi Kasei, Inc. | 0.06 | 0.06 | 0.06 | 0.06 | 0.06 |
| | Dimethicone | XIAMETERTM PMX-200 5cs | 5.45 | 5.45 | 5.45 | 5.45 | 5.45 |
| C | Sodium chloride | | 1 | 1 | 1 | 1 | 1 |
| | Glycerin | | 5 | 5 | 5 | 5 | 5 |
| | Water | | Residual | Residual | Residual | Residual | Residual |
| D | Preservative | Euxyl PE9010 available from Schülke & Mavr | 0.7 | 0.7 | 0.7 | 0.7 | 0.7 |
| Evaluation of tactile sensation | | | Poor | Poor emulsification | △ | Good | Poor |

### (Method of preparation)

1. Phase A is mixed.
2. Phase B is mixed.
3. Phase B is slowly added while stirring Phase A.
4. Phase C is added to 3 above and then stirred until uniform.

As shown in Table 5, the oil-in-water sunscreen agent using the silicone elastomer particles of the present invention (Examples 7 and 8) was evaluated to have favorable spreadability, a smooth and moist feel, and there was no squeaky feel, unlike a case which did not contain elastomer particles (Comparative Example 4), as well as sunscreen using other particles (Comparative Example 6). In Comparative Example 5, the DOWSIL^{™} EP-9610 Cosmetic Powder absorbed the oil (dimethicone) and emulsifier (DOWSIL^{™} ES-5612 Formulation Aid), and emulsification was not possible.

### [Examples 9 and 10 and Comparative Examples 7 and 8]

The panelists compared and evaluated the feel during use of water-in-oil skin cream in which the silicone elastomer particles in the compositions listed in Table 7 were used.

### Evaluation of Tactile Sensation

The 18 panelists evaluated the spreadability and white residue when the samples were applied to inner forearms using the criteria in Table 6 below.

**[Table 6]**

| Evaluation results | Evaluation Indicators |
|---|---|
| Good | 12 or more of 18 persons responded favorable |
| △ | 7 to 11 of 18 persons responded favorable |
| Poor | 6 or fewer of 18 persons responded favorable |

**[Table 7]**

| Phase | Component | Product name and supplier | Comparative Examples 7 | EXAMPLES 9 | EXAMPLES 10 | Comparative Examples 8 |
|---|---|---|---|---|---|---|
| A | Silicone emulsifier premix | ACULYN^{™} Siltouch Rheology Modifier | 2 | 2 | 2 | 2 |
| | Glyceryl tri(capric acid/caprylic acid) | | 2 | 2 | 2 | 2 |
| | Almond oil | | 6 | 6 | 6 | 6 |
| | Silicone elastomer particle No. 1 | (Example 3) | | 1 | | |
| | Silicone elastomer particle No. 2 | (Example 4) | | | 1 | |
| | Particles of Comparative Example 1 | (Comparative Example 1) | | | | 1 |
| B | BG (Butylene glycol) | | 5 | 5 | 5 | 5 |
| | Water | | Residual | Residual | Residual | Residual |
| C | Preservative | Euxyl PE9010 available from Schülke & Mayr | 0.7 | 0.7 | 0.7 | 0.7 |
| Evaluation of tactile sensation | | | Poor | Good | Good | △ |

As shown in Table 7, the water-in=-oil skin cream using the silicone elastomer particles of the present invention (Examples 9 and 10) was evaluated to have favorable spreadability, a smooth and moist feel, and there was no squeaky feel, unlike a case which did not contain elastomer particles (Comparative Example 7), as well as skin cream using other particles (Comparative Example 8).

### [Synthesis Example 11: Acryl-modified polycarbonate compound b 3]

The same operation as Example 1 was performed except for using 26.7 parts by weight of a polycarbonate expressed by the following structural formula (1): 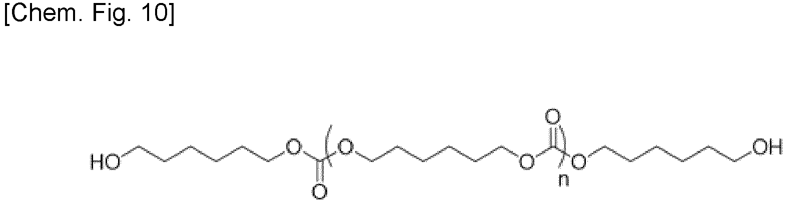 (Mw=500; n ≧ 0 in the formula)
(UH-50, ETERNACOLL(R) UH series, manufactured by UBE Corporation), 26.7 parts by weight of chloroform, 36.9 parts by weight of potassium carbonate, 63 ppm of MEHQ (hydroquinone monomethyl ether, polymerization inhibitor), and 9.7 parts by weight of acryloyl chloride, to obtain a polycarbonate terminated with an acryl group 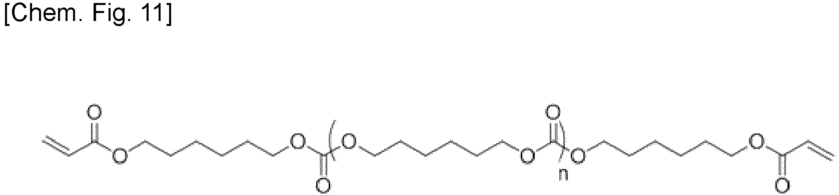 ((meth)acryl-modified polycarbonate compound) (b3).
The acryl group-modified polycarbonate (b3) can be used as a raw material for producing silicone elastomer particles, similar to component (b1) or component (b2) in Example 3 (hydrosilylation reaction type) or Example 4 (radical polymerization reaction type).

## Claims

1. A reactive group-containing polycarbonate compound having in each molecule 2 or more modified polycarbonate structures expressed by the following structural formula (1): 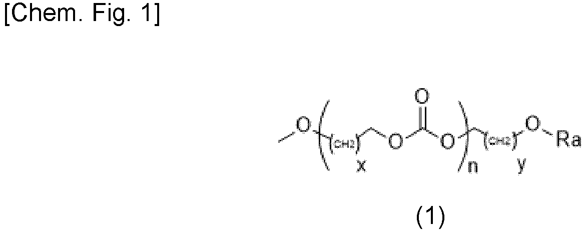 {where x and y are both numbers ranging from 0 to 18, n is a number ranging from 0 to 30, and Ra is a reactive group selected from (meth)acryl terminal groups expressed by -C(=O)-R¹-CR²=CH₂ (R¹ is a chemical bond between CH and C(=O) or a divalent organic group having 0 to 20 carbon atoms, and R² is a hydrogen atom or a methyl group) and alkenyl terminal groups having 2 to 20 carbon atoms}.

2. The reactive group-containing polycarbonate compound according to claim 1, wherein in the modified polycarbonate structure expressed by structural formula (1), x is a number ranging from 1 to 18.

3. The reactive group-containing polycarbonate compound according to claim 1, wherein in the functional group Ra in structural formula (1), R¹ is a chemical bond between an oxygen atom (O) and C(=O) or an alkylene group having 1 to 20 carbon atoms.

4. The reactive group-containing polycarbonate compound according to any one of claims 1 to 3, expressed by structural formula (1-1): 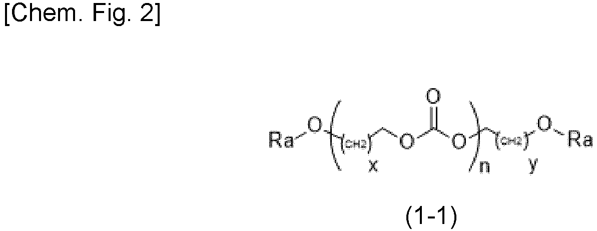 (where Ra is the same group as defined above, and x, y, and n are the same numbers as defined above).

5. The reactive group-containing polycarbonate compound according to any one of claims 1 to 4, which is a synthesis raw material for silicone elastomer particles.

6. A method for producing a reactive group-containing polycarbonate compound according to any one of claims 1 to 4, the method comprising:
reacting a polycarbonate compound expressed by the following structural formula (1'): 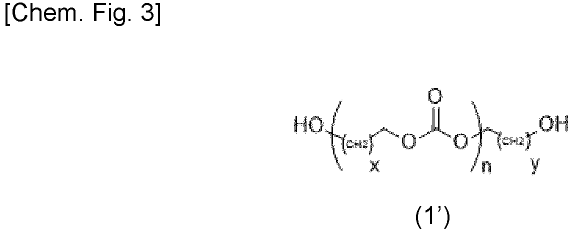
(where x, y, and n are the same numbers as defined above),
and
a (meth)acryloyl chloride compound expressed by Cl-C(=O)-R¹-CR²=CH₂ (where R¹ and R² are the same groups as defined above),
in the presence of a basic catalyst.

7. Silicone elastomer particles having a structure in which at least two silicon atoms in the silicone elastomer particle are crosslinked by one or more type of reaction selected from a radical polymerization reaction of the reactive group-containing polycarbonate compound according to any one of claims 1 to 4, and a hydrosilylation reaction with a silicon atom-bonded hydrogen atom.

8. The silicone elastomer particles according to claim 7, further comprising in the silicone elastomer particles a polyorganosiloxane structure expressed by:
-(R₂SiO)ₘ-
(where R represents an unsubstituted alkyl group having 1 to 20 carbon atoms or an alkyl group having 1 to 20 carbon atoms substituted with a halogen atom, an aryl group having 6 to 22 carbon atoms, or a hydroxyl group, and m is a number ranging from 1 to 1000).

9. The silicone elastomer particles according to claim 7 or 8, wherein the silicone elastomer particles include at least:
(A) at least one type of reactive organopolysiloxane selected from the following component (a1) and component (a2);
(a1) an organopolysiloxane having at least three organic groups containing a (meth)acryloxy group of at least one type selected from organic groups containing a methacryloxy group and organic groups containing an acryloxy group in each molecule;
(a2) an organopolysiloxane having at least three silicon atom-bonded hydrogen atoms per molecule;
(B) a reactive group-containing polycarbonate compound according to any one of claims 1 to 4; and
(C) one or more curing agent selected from radical polymerization initiators and hydrosilylation reaction catalysts; and are obtained by performing in water a crosslinking reaction of crosslinkable silicone emulsified particles obtained by emulsifying in water a crosslinkable silicone composition crosslinkable by one or more reactions selected from radical polymerization reactions and hydrosilylation reactions with a silicon-bonded hydrogen atom.

10. The silicone elastomer particles according to any one of claims 7 to 9, wherein the average primary particle diameter as measured by a laser diffraction scattering method is 0.5 to 20 µm.

11. The silicone elastomer particles according to any one of claims 7 to 10, wherein the crosslinking reactive silicone composition used in forming the silicone elastomer particles is cured in a sheet form, and the JIS-A hardness measured is within a range of 10 to 80.

12. The silicone elastomer particles according to any one of claims 7 to 10, further comprising a structure in which some or all of the surface thereof is covered with one or more types selected from organopolysiloxane resins, silica, and other silicone elastomer particles.

13. The silicone elastomer particles according to any one of claims 7 to 10, further comprising a mesoporous structure.

14. The silicone elastomer particles according to any one of claims 7 to 10, further comprising an oil agent that is liquid at 40°C.

15. The silicone elastomer particles according to any one of claims 7 to 14, having biodegradability.

16. The silicone elastomer particles according to any one of claims 7 to 14, wherein a divalent organic group having a partial structure formed by radical polymerization or hydrosilylation reaction of a reactive group-containing polycarbonate compound is active with respect to biodegradable reactions, and in a biodegradable environment, a crosslinked structure formed between silicon atoms in the silicone elastomer particle is at least partially cleaved, and primary particles of the silicone elastomer particles have a property of disintegrating with the generation of a polyorganosiloxane with a non-crosslinked structure.

17. A cosmetic material raw material, comprising the silicone elastomer particles according to any one of claims 7 to 16.

18. A cosmetic material composition, comprising the silicone elastomer particles according to any one of claims 7 to 16.

19. An organic resin additive, comprising the silicone elastomer particles according to any one of claims 7 to 16.

20. An organic resin, comprising the silicone elastomer particles according to any one of claims 7 to 16.

21. A method of manufacturing the silicone elastomer particles according to any one of claims 7 to 16, comprising the following step (I) and step (II).
Step (I): a step of forming crosslinkable emulsified particles, by emulsifying in water
(A) at least one type of reactive organopolysiloxane selected from the following component (a1) and component (a2);
(a1) an organopolysiloxane having at least three organic groups containing a (meth)acryloxy group of at least one type selected from organic groups containing a methacryloxy group and organic groups containing an acryloxy group in each molecule;
(a2) an organopolysiloxane having at least three silicon atom-bonded hydrogen atoms per molecule;
(B) a reactive group-containing polycarbonate compound according to any one of claims 1 to 4; and
(C) one or more curing agents selected from radical polymerization initiators and hydrosilylation reaction catalysts;
Step (II): a step of curing the crosslinking reactive silicone emulsified particle obtained in step (I) in the presence of (C) a curing agent to obtain silicone elastomer particles.
